# EUROPÄISCHE PATENTANMELDUNG

(11) **EP 1 946 736 A1**
(43) Veröffentlichungstag der Anmeldung: **23.07.2008**
(21) Anmeldenummer: 08100596.9
(22) Anmeldetag: 17.01.2008
(51) Int. Cl.: A61H 1/00, A61H 23/02

(54) **Vibrationstherapievorrichtung**

(30) Priorität: 17.01.2007 DE 102007003361
(71) Anmelder: Patex Group Ltd., Tortola, Virgin Islands, British (VG)
(72) Erfinder: Guenther, Andreas, 98000, Monaco (MC); Vogt, Thomas, 9494, Schaan (LI)
(74) Vertreter: Freischem, Stephan

(57) **Zusammenfassung**

Die Erfindung betrifft eine Vorrichtung zur Übertragung von Schwingungen auf eine Person, mit einem Tragelement (1) zur Aufnahme eines menschlichen Körpers und mit mindestens einem auf der Rückseite des Tragelements (1) angeordneten Schwingungsgenerator(4,5,6), der mittels eines Elektromagneten Schwingungen erzeugt.

Aufgabe der vorliegenden Erfindung ist es, die positive Wirkung der gattungsgemäßen Vorrichtung auf das Wohlbefinden eines Menschen zu steigern. Diese Aufgabe wird erfindungsgemäß dadurch gelöst, dass zwischen dem Tragelement (1) und dem Elektromagneten eine Abschirmung (7) angeordnet ist, die aus einem Magnetfelder abschirmenden Material besteht, und dass zwischen der Abschirmung (7) und dem Tragelement (1) oder auf dem Tragelement (1) mindestens ein elektrischer Leiter zum Erzeugen eines Magnetfeldes angeordnet ist.

## Beschreibung

### Technisches Gebiet

Die Erfindung betrifft eine Vorrichtung zur Übertragung von Schwingungen auf eine Person, mit einem Tragelement zur Aufnahme eines menschlichen Körpers und mit mindestens einem an der Rückseite des Tragelements angeordneten Schwingungsgenerator, der mittels eines Elektromagneten Schwingungen erzeugt.

### Stand der Technik

Derartige Vorrichtungen können allgemein als Vibrationstherapievorrichtung oder Klangtherapievorrichtung bezeichnet werden. Sie sind im Wesentlichen wie ein Sitzmöbel oder Liegemöbel gestaltet, wobei das Tragelement vorzugsweise eine Sitzfläche oder Liegefläche bildet. Die Vorrichtungen umfassen elektromagnetische Wandler, welche elektrische Ströme in Vibrationen oder Schall umwandeln. Aus der Druckschrift DE 26 14 972 ist eine derartige Vorrichtung bekannt, bei der einerseits durch einen elektroakustischen Wandler Schallwellen erzeugt werden und andererseits ein elektromechanischer Wandler niederfrequente Vibrationsschwingungen erzeugt.

Weitere Vorrichtungen dieser Art gehen aus den Druckschriften DE 202 08 228 U1, DE 35 22 305 C2, DE 201 13 443 U1, EP 0 441 802 B1, EP 1 491 228 A2, EP 0 244 508 B1, EP 0 224 102 B1, WO 2005/030011 A1, EP 0 422 253 A1, EP 0 352 569 B1, EP 0 296 231 B1, EP 1 325 727 A2 und WO 2005/020869 A1 bekannt.

Die unterschiedliche Natur der auf den menschlichen Körper übertragenen Schwingungen geht gut aus der genannten DE 26 14 972 C3 hervor. Während die elektroakustischen Wandler wie Lautsprecher über eine Membran Schwingungen im hörbaren Frequenzbereich erzeugen, übertragen elektromagnetische Schwingungserzeuger (in Englisch: vibrotactile transducer) über eine starre Kopplung mechanische Vibrationen oder Schwingungen unmittelbar auf das Tragelement.

Es hat sich gezeigt, dass die Übertragung von Schwingungen (Schallwellen oder Vibrationen) auf einen Menschen, der in einer entspannten Haltung auf einem Tragelement ruht, eine beruhigende und entspannende Wirkung auf den Menschen ausübt. Vorzugsweise werden Schallwellen und Vibrationen gleichzeitig auf den menschlichen Körper übertragen. Die Kombination der beiden unterschiedlichen Signale erhöht die entspannende Wirkung.

Druckschriften, die eine Kombination einer Vorrichtung zur Schalltherapie oder Vibrationstherapie vorschlagen, sind z.B. die US 2005/0154249 A1, DE 101 09 429 A1, DE 100 60 306 A1, DE 44 31 661 A1, US 4,326,506

Die US 2005/0154249 A1 schlägt Permanentmagneten zur Erzeugung eines Magnetfeldes vor. Hierdurch können keine zeitlich variablen Magnetfelder erzeugen, die sich in der Magnetfeldtherapie als besonders effektiv erwiesen haben. Die weiter oben genannte Druckschrift DE 101 09 429 A1 beschreibt ein therapeutisches Sitz- und/oder Liegemöbel mit einer Sitz- und/oder Liegefläche, der wenigstens eine Magnetfeldspule zugeordnet ist, die von einem Verstärker mit einem zeitlich veränderlichen Signal zur Erzeugung eines zeitvariablen Magnetfeldes beaufschlagt ist. Um eine Erweiterung der therapeutischen Anwendungsmöglichkeiten zuzulassen, wird vorgeschlagen, dass die wenigstens eine Spule Bestandteil eines elektroakustischen Wandlers ist und die Sitz- und/oder Liegefläche mittels des elektroakustischen Wandlers zur Abstrahlung von Schallschwingungen angeregt wird. Bei dieser Vorrichtung wird also das das zeitvariable Magnetfeld, dass auf einen auf der Sitz/Liegefläche liegenden Menschen wirkt, gleichzeitig zur Erzeugung der Schallschwingungen verwendet. Die Schallschwingungen müssen zwingend synchron zu den Fluktuationen des auf den Menschen wirkenden Magnetfeldes sein. Auch in den Druckschriften DE 196 05 777 A1 und US 4,326,506 wird vorgeschlagen, das Magnetfeld der Spule des Vibrationserzeugers auf den Körper wirken zu lassen.

### Darstellung der Erfindung

Aufgabe der vorliegenden Erfindung ist es, die gattungsgemäße Vorrichtung derart weiterzubilden, dass deren positive Wirkung auf das Wohlbefinden eines Menschen gesteigert werden kann.

Diese Aufgabe wird erfindungsgemäß dadurch gelöst, dass zwischen dem Tragelement und dem Elektromagneten eine Abschirmung angeordnet ist, die aus einem Magnetfelder abschirmenden Material besteht, und dass zwischen der Abschirmung und dem Tragelement oder auf dem Tragelement mindestens ein elektrischer Leiter zum Erzeugen eines Magnetfeldes angeordnet ist.

Mit anderen Worten wird vorgeschlagen, eine bekannte Vorrichtung zur Übertragung von Schwingungen auf den menschlichen Körper mit einer Magnetfeldtherapievorrichtung zu kombinieren, wobei die Magnetfelder der Schwingungsgeneratoren von den auf den menschlichen Körper wirkenden Magnetfeldern entkoppelt sind. Zu diesem Zweck werden die Magnetfelder, welche durch den Elektromagneten des Schwingungsgenerators (Lautsprecher und/oder Vibrationsgenerator) erzeugt werden, abgeschirmt. Eine das Magnetfeld schwächende Abschirmung wird zwischen dem Tragelement und dem Elektromagneten angeordnet. Hierfür eignet sich ein ferromagnetisches Material wie Eisen. Gleichzeitig wird oberhalb der Abschirmung, d.h. zwischen der Abschirmung und dem Tragelement oder auf dem Tragelement, mindestens ein elektrischer Leiter zum Erzeugen eines definierten Magnetfeldes zur Durchführung einer Magnetfeldtherapie angeordnet.

Magnetfeldtherapievorrichtungen sind beispielsweise bekannt aus der Druckschrift DE 103 04 085 A1. Sie weisen beispielsweise mattenartige Vorrichtungen auf, in denen elektrische Leiterbahnen aufgenommen sind. Die elektrischen Leiterbahnen sind über Anschlusskabel und Steckverbinder mit einem Steuergerät verbunden. Über das Steuergerät wird ein Strom in die elektrischen Leiterbahnen der Magnetfeldmatte eingeleitet, der ein Magnetfeld über der Magnetfeldmatte erzeugt. Der Strom ist zeitlich variabel und verläuft vorzugsweise in Einzelimpulsen, die zu Impulsgruppen zusammengefasst sind, welche jeweils durch Pausen zwischen zwei Impulsgruppen getrennt sind. Die Form und Frequenz der einzelnen Stromimpulse, der zeitlich variable Amplitudenverlauf der Stromimpulse sowie die Pause zwischen den aufeinander folgenden Impulsgruppen haben wesentlichen Einfluss auf die Wirkung des Magnetfelds auf den Organismus des Patienten. Diese Größen können bei der Magnetfeldtherapievorrichtung der genannten Druckschrift in Abhängigkeit von einem Wert gesteuert werden, der durch einen Biosensor (Pulssensor, Blutdrucksensor etc.) erfasst wird.

Die magnetischen Schwingungen, die von den Schwingungsgeneratoren beim Erzeugen der Schwingungen entstehen, haben in aller Regel eine Form, die von den optimalen zeitlichen Fluktuationen des durch den Leiter generierten und auf den menschlichen Körper wirkenden Magnetfeldes abweicht.

Um die ordnungsgemäße Funktion der durch die Leiterbahnen realisierten Magnetfeldtherapievorrichtung zu gewährleisten, ist es erforderlich, störende oder parasitäre Magnetfelder, insbesondere die von dem Elektromagneten des Schwingungsgenerators ausgehenden Magnetfelder, gegenüber dem auf dem Tragelement befindlichen menschlichen Körper abzuschirmen. So ist sichergestellt, dass allein die therapeutischen Magnetfelder, welche durch die Leiterbahn erzeugt werden, auf den menschlichen Körper einwirken und den beabsichtigten therapeutischen Effekt erzielen. Die auf den Menschen wirkenden Magnetfelder können zwar mit den Magnetfeldern für die Schwingungserregung synchronisiert werden, müssen aber nicht synchron sein. Durch die Abschirmung können beide Magnetfelder unabhängig von einander erzeugt werden und beeinflussen sich nicht gegenseitig.

Der Elektromagnet kann in der Praxis in einem Gehäuse angeordnet sein, welches aus einem Magnetfelder abschirmenden Material besteht und die Abschirmung bildet.

Alternativ oder zusätzlich kann an oder unter dem Tragelement eine Platte aus einem Magnetfelder abschirmenden Material angeordnet sein. Mit derartigen Platten lassen sich bereits sehr wirksame Reduktionen der oberhalb des Tragelements einwirkenden magnetischen Feldstärke erzielen. Die Kombination beider Abschirmungen (Gehäuse und abschirmende Platte) bewirkt eine noch stärkere Reduktion der parasitären Magnetfelder, ist aber aufwändiger und schwerer.

Die magnetische Abschirmung besteht - wie oben erwähnt - vorzugsweise aus einem elektrisch leitfähigen und ferromagnetischen Material, insbesondere aus Eisen. Die elektrische Leitfähigkeit wirkt den hochfrequenten Magnetfeldern entgegen. Die ferromagnetische Eigenschaft schirmt niederfrequente Magnetfelder z.B. mit Netzfrequenz (50-60 Hz) ab.

Der elektrische Leiter zum Erzeugen eines oberhalb des Tragelements auf den menschlichen Körper einwirkenden Magnetfeldes kann auf einer Oberfläche des Tragelements aufgebracht sein. Der elektrische Leiter kann insbesondere in eine mattenartige Struktur aus polsterndem Material integriert sein, welche auf die Oberseite des Tragelements aufgelegt wird.

Bei einer bevorzugten Ausführungsform ist der elektrische Leiter jedoch fest mit einer Oberfläche des Tragelements verbunden. Der elektrische Leiter kann beispielsweise auf die obere oder untere Oberfläche des Tragelements aufgeklebt sein. Die feste Verbindung des Leiters mit dem Tragelement hat den Vorteil, dass der Leiter in die Gesamtstruktur der Therapievorrichtung integriert ist. Bei einer besonderen Ausführungsform ist der elektrische Leiter in das Tragelement eingebettet. Hierzu kann das Tragelement zumindest teilweise aus Kunststoffmaterial bestehen, in welches der elektrische Leiter integriert ist. Beispielsweise kann das Tragelement von einem plattenartigen Bauteil gebildet werden, wobei der elektrische Leiter auf eine Oberfläche dieses Bauteils aufgelegt und hiermit durch ein Kunstharz wie Epoxidharz fest verbunden ist. Das Kunstharz oder Epoxidharz bildet nach dem Aushärten eine geschlossene Kunststoffschicht, in die der elektrische Leiter integriert ist.

Insbesondere wenn das plattenartige Bauteil aus hochwertigem Material besteht (z.B. Holz), kann der elektrische Leiter auf die beschriebene Weise an der unteren Oberfläche des plattenförmigen Bauelements angebracht werden. Der elektrische Leiter kann aber auch auf die obere Oberfläche aufgelegt und hiermit vergossen werden. Hierbei wird ein transparentes Epoxidharz verwendet, wenn die Struktur des Tragelements, einschließlich des darin integrierten Leiters, sichtbar sein soll.

Der elektrische Leiter kann in der Praxis die Form einer ebenen Spule aufweisen, also nach Art einer Spirale verlaufen. Eine bevorzugte Form der Anordnung des elektrischen Leiters im Bereich der Liegefläche ist beispielsweise in der Druckschrift DE 103 04 093 A1 beschrieben.

Bei einer bevorzugten Ausführungsform kann die Vorrichtung ein schalenartiges Gestell aufweisen, welches eine obere Öffnung bildet, die durch das flächige Tragelement abgedeckt ist. Diese schalenartige Ausgestaltung verleiht der Vorrichtung einen Resonanzkörper. Das schalenartige Gestell bildet zusammen mit dem die obere Öffnung verschließenden flächigen Tragelement einen Resonanzkörper, der Schwingungen verstärkt, wenn diese mittels des Elektromagneten in das Tragelement eingeleitet werden. Hierdurch kann die zur Erzeugung der Schwingungen erforderliche Leistung reduziert werden.

Die therapeutische Wirkung der erfindungsgemäßen Vorrichtung kann ferner dadurch erhöht werden, dass oberhalb des Tragelements mindestens eine Lichtquelle angeordnet ist. Durch die Lichtquelle kann der Kopfbereich der auf dem Tragelement liegenden Person beleuchtet werden. Vorzugsweise ist die Farbe und/oder die Strahlungsintensität der Lichtquelle steuerbar. Die Vibrationstherapie oder Klangtherapie kann folglich nicht nur mit einer Magnetfeldtherapie, sondern auch einer Lichttherapie gekoppelt werden. Dabei können Lichtimpulse oder Farbwechsel auf die durch die Vorrichtung erzeugten Schwingungen und therapeutischen Magnetfelder abgestimmt werden.

### Kurze Beschreibung der Zeichnungen

Ausführungsformen der Erfindung werden nachfolgend unter Bezugnahme auf die beigefügten Zeichnungen beschrieben. Die Zeichnungen zeigen in:
- Fig. 1: eine schematische Schnittansicht einer erfindungsgemäßen Vorrichtung,
- Fig. 2: eine schematische Unteransicht eines Abschnitts des Schwindungsgenerators der erfindungsgemäßen Vorrichtung,

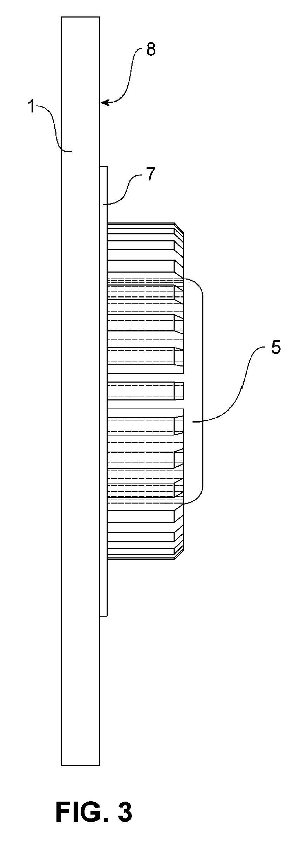

- Fig. 3: die Seitenansicht des Schwindungsgenerators aus Fig. 2,
- Fig. 4: eine Draufsicht auf das Tragelement der erfindungsgemäßen Vorrichtung,
- Fig. 5: eine schaubildliche Darstellung eines Gestells mit Tragelement für die erfindungsgemäße Vorrichtung in Explosionsansicht und
- Fig. 6: eine schaubildliche Gesamtansicht der erfindungsgemäßen Vorrichtung aus Fig. 5 mit Lichtquelle für die Lichttherapie.

### Ausführungsformen der Erfindung

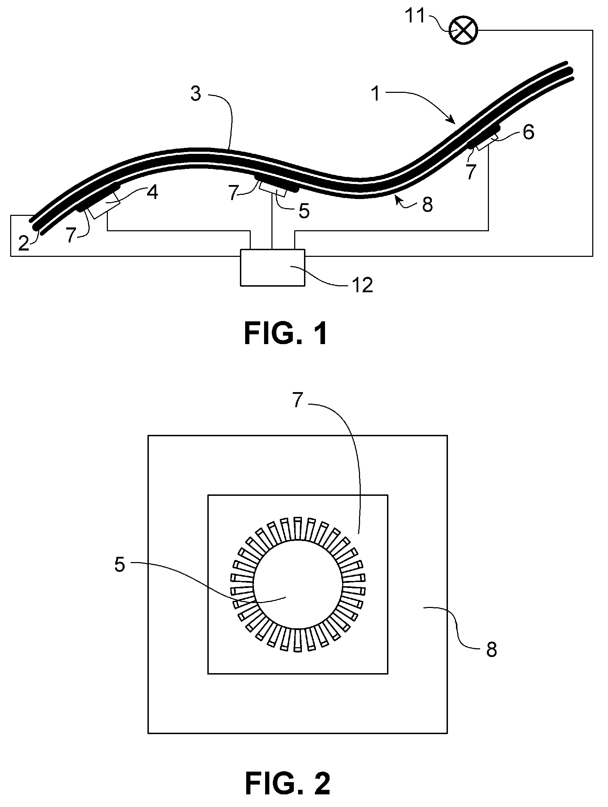

Die in der Fig. 1 dargestellte Vorrichtung zur Übertragung von Schwingungen auf einen menschlichen Körper besteht aus einem Tragelement 1, das im vorliegenden Fall als Liegefläche ausgebildet ist. Das Tragelement 1 umfasst in der schematischen Darstellung einen harten Kern 2 und eine polsternde Auflage 3. An der Unterseite oder Rückseite des Kerns 2 sind Schwingungsgeneratoren 4,5,6 angeordnet, in denen ein Elektromagnet auf bekannte Weise mechanische Schwingungen erzeugt, welche entweder als Vibrationen in das Tragelement 1 eingeleitet werden oder über Membranen Schallwellen erzeugen.

Zwischen jedem Schwingungsgenerator 4,5,6 und dem Tragelement 1 ist eine plattenförmige Abschirmung 7 vorgesehen, welche im vorliegenden Fall aus einer Eisenplatte besteht. Eisen ist ein magnetisch leitendes Material und schirmt den Bereich oberhalb des Tragelements 1 gegenüber der magnetischen Strahlung der Schwingungsgeneratoren 4,5,6 ab.

Die Rückseite eines Schwingungsgenerators 5 ist vergrößert in der Fig. 2 dargestellt. Die Abschirmungsplatte 7 aus Eisen reduziert erheblich die magnetische Feldstärke des von den Elektromagneten der Schwingungsgeneratoren 4,5,6 erzeugten Magnetfeldes im Bereich des oberhalb des Trag-elements 1.

Alternativ könnte eine durchgehende Eisenplatte an der Rückseite des Tragelements 1 vorgesehen werden. Dies hätte allerdings ein erheblich höheres Gewicht zur Folge.

In Fig. 1 sind neben den Schallgeneratoren 4,5,6 weitere Signalerzeuger zu erkennen, welche auf einen auf dem Tragelement 1 liegenden menschlichen Körper therapeutisch wirken. Oberhalb eines Bereichs des Tragelements 1, in dem sich der Kopf der auf dem Tragelement 1 liegenden Person befindet, ist eine Lichtquelle 11 dargestellt. Die Lichtquelle 11 ist vorzugsweise in ihrer Helligkeit und Farbe steuerbar, so dass die Lichtsignale auf die erzeugten Schwingungen und den magnetischen Wechselfeldern abgestimmt sind, welche auf die auf dem Tragelement 1 liegende Person einwirken.

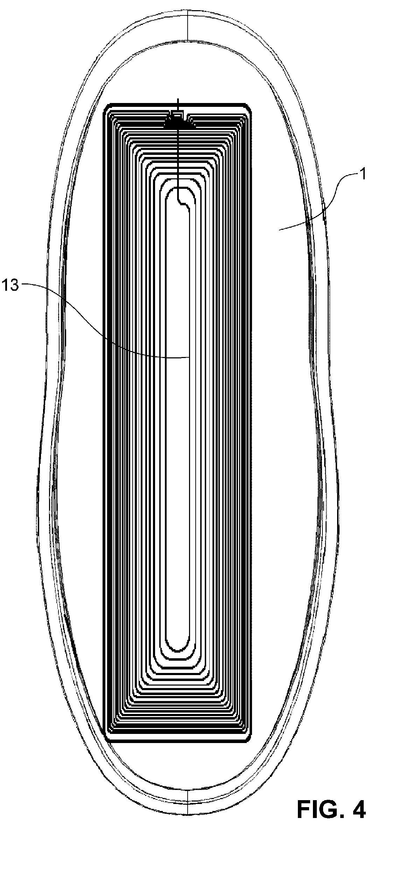

Die Schallgeneratoren 4,5,6 sowie die Lichtquelle 11 werden durch eine gemeinsame Steuerungsanordnung 12 angesteuert und betrieben. Die Steuerungsanordnung 12 ist über einzelne Signalleitungen mit den Schallgeneratoren 4,5,6 und der Lichtquelle verbunden. Eine weitere Signalleitung führt von der Steuerungsanordnung 12 zu dem Tragelement 1. Diese weitere Signalleitung führt einem elektrischen Leiter im Bereich des Tragelements 1 ein elektrisches Signal zu, welches ein dynamisches Magnetfeld oberhalb des Tragelements 1 erzeugt. Eine Ausführungsform des elektrischen Leiters ist genauer in Fig. 4 dargestellt.

Die Fig. 4 zeigt eine Draufsicht auf das Tragelement 1, welches im Schnitt in Fig. 1 dargestellt ist. Zum Tragen des Gewichts der aufliegenden Person kann das Bauelement einen harten Kern 2, z.B. aus Holz oder Kunststoff, aufweisen. Auf der Oberseite des Tragelements ist ein elektrischer Leiter 13 vorgesehen, der sich im Wesentlichen über die gesamte Länge und einen großen Teil der Breite des Tragelements 1 in Form einer rechteckigen Spirale erstreckt. Die Leiterspirale kann aber auch andere Formen aufweisen, z.B. gerundet sein. Außerdem kann der Leiter anderen Bahnen z.B. wellenartigen oder mäanderförmigen Bahnen folgen.

Der elektrische Leiter 13 dient der Erzeugung eines Magnetfelds. Es werden ihm dynamische elektrische Ströme in Impulsen zugeführt, welche ein magnetisches Wechselfeld mit vorgegebener Impulsform und vorgegebener Stärke oberhalb des Tragelements 1 der Vorrichtung erzeugen. Dieses magnetische Wechselfeld wird nicht durch die Magnetfelder der Schwingungsgeneratoren 4,5,6 gestört, da diese Magnetfelder mittels der Abschirmungen 7 gegenüber dem Bereich oberhalb des Tragelements 1 abgeschirmt sind.

In der dargestellten Ausführungsform ist der Leiter 13 fest mit dem Tragelement 1 verbunden. Er ist hierzu auf den Kern 2 aufgelegt und mit einem transparenten Kunststoffharz vergossen. Diese Vorgehensweise führt zu einem sehr robusten Gerät, bei dem die Vorrichtung zur Erzeugung des Magnetfelds unmittelbar in dem Tragelement 1 integriert ist. Die Kunststoffschicht bildet die Oberseite des Tragelements 1 und ist transparent.

Wie in Verbindung mit Fig. 1 erläutert, ist der elektrische Leiter 13 über eine Signalleitung und entsprechende Verbindungskontakte mit der Steuerungsanordnung 12 verbunden. Die Steuerungsanordnung 12 ist in der Regel ein elektronischer Signalgenerator, der die zur Ansteuerung der verschiedenen Einrichtungen erforderlichen Ströme erzeugt. Die Steuerungsanordnung 12 ist hier als Bedienpult ausgebildet und weist Bedienelemente auf, die der Benutzer der Vorrichtung betätigen kann. Durch die Integration des elektrischen Leiters 13 in die erfindungsgemäße Vorrichtung und dessen Abschirmung gegenüber den Elektromagneten der Schallgeneratoren und Schwingungsgeneratoren 4,5,6 wird eine variationsreichere und effektivere Behandlung einer auf dem Tragelement 1 aufliegenden Person ermöglicht, wobei das durch den Leiter 13 erzeugte Magnetfeld nicht durch parasitäre Magnetfelder gestört oder verfälscht wird. Eine weitere Variante der therapeutischen Beeinflussung kann durch die Lichtquelle 11 erzielt werden.

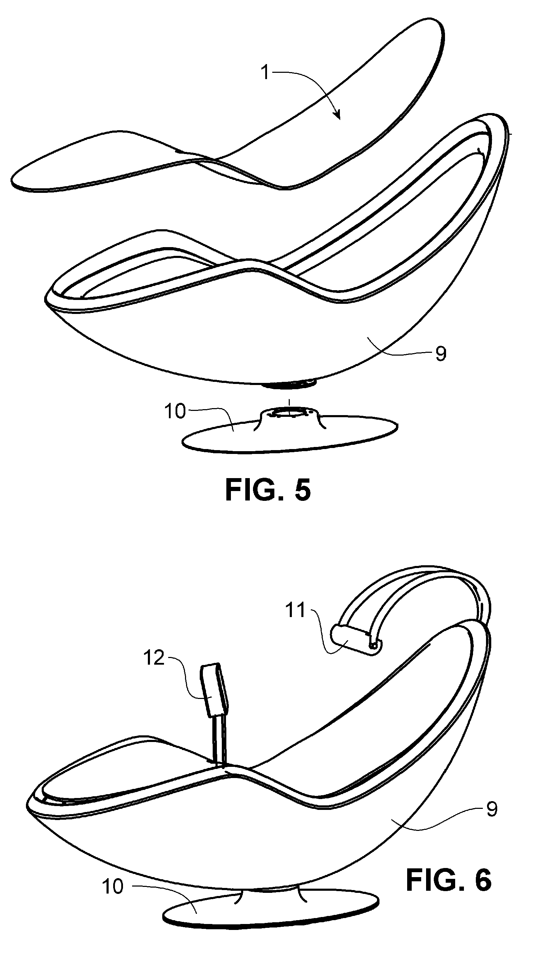

Eine praktische Ausführungsform der erfindungsgemäßen Vorrichtung ist in den schaubildlichen Darstellungen der Fig. 5 und 6 dargestellt. Es ist zu erkennen, dass die Vorrichtung ein schalenartiges Gestell 9 aufweist, welches etwa die Form und Dimension einer Badewanne hat. Das schalenartige Gestell 9 bildet eine obere Öffnung, welche durch das Tragelement 1 abgedeckt wird. Das schalenartige Gestell 9 kann aus zwei Halbschalen bestehen. Das schalenartige Gestell 9 wird vorzugsweise aus dünnwandigem Kunststoffmaterial, ggf. mit Faserverstärkung gefertigt. Das schalenartige Gestell 9 wird auf einem Fuß 10 befestigt.

Das schalenartige Gestell 9 bildet zusammen mit dem Tragelement 1 einen Resonanzkörper, der die durch die Schwingungsgeneratoren 4,5,6 induzierten Schwingungen verstärkt.

Wie bereits erwähnt, können die Schwingungsgeneratoren 4,5,6 Schallwellen oder Vibrationen des Tragelements 1 erzeugen.

Die Fig. 6 zeigt eine schaubildliche Seitenansicht der zusammengesetzten erfindungsgemäßen Vorrichtung. Hier ist auch ein Träger für die Lichtquelle 11 oberhalb des Kopfbereichs der auf dem Tragelement 1 liegenden Person sowie eine Steuerungsanordnung 12 zu erkennen, mit der die erfindungsgemäße Vorrichtung bedient werden kann.

Bezugszeichenliste:
- 1: Tragelement
- 2: Kern
- 3: Auflage
- 4: Schwingungsgenerator
- 5: Schwingungsgenerator
- 6: Schwingungsgenerator
- 7: Abschirmung
- 8: Rückseite
- 9: schalenartiges Gestell
- 10: Fuß
- 11: Lichtquelle
- 12: Steuerungsanordnung
- 13: elektrischer Leiter

## Patentansprüche

1. Vorrichtung zur Übertragung von Schwingungen auf eine Person, mit einem Tragelement (1) zur Aufnahme eines menschlichen Körpers und mit mindestens einem auf der Rückseite des Tragelements (1) angeordneten Schwingungsgenerator(4,5,6), der mittels eines Elektromagneten Schwingungen erzeugt, **dadurch gekennzeichnet, dass** zwischen dem Tragelement (1) und dem Elektromagneten eine Abschirmung (7) angeordnet ist, die aus einem Magnetfelder abschirmenden Material besteht, und dass zwischen der Abschirmung (7) und dem Tragelement (1) oder auf dem Tragelement (1) mindestens ein elektrischer Leiter (13) zum Erzeugen eines Magnetfeldes angeordnet ist.

2. Vorrichtung nach Anspruch 1, **dadurch gekennzeichnet, dass** der Elektromagnet in einem Gehäuse angeordnet ist und das Gehäuse aus einem Magnetfelder abschirmenden Material besteht.

3. Vorrichtung nach Anspruch 1 oder 2, **dadurch gekennzeichnet, dass** an oder unter dem Tragelement (1) eine Platte (7) aus einem Magnetfelder abschirmenden Material angeordnet ist.

4. Vorrichtung nach einem der vorangehenden Ansprüche, **dadurch gekennzeichnet, dass** das Magnetfelder abschirmende Material ein ferromagnetisches Material ist.

5. Vorrichtung nach einem der vorangehenden Ansprüche, **dadurch gekennzeichnet, dass** das Magnetfelder abschirmende Material Eisen ist.

6. Vorrichtung nach einem der vorangehenden Ansprüche, **dadurch gekennzeichnet, dass** der elektrische Leiter (13) zum Erzeugen eines Magnetfeldes auf eine Oberfläche des Tragelements (1) aufgebracht, insbesondere aufgeklebt ist.

7. Vorrichtung nach einem der vorangehenden Ansprüche, **dadurch gekennzeichnet, dass** der elektrische Leiter (13) in das Tragelement (1) eingebettet ist.

8. Vorrichtung nach Anspruch 7, **dadurch gekennzeichnet, dass** das Tragelement (1) aus Kunststoffmaterial besteht.

9. Vorrichtung nach einem der vorangehenden Ansprüche, **dadurch gekennzeichnet, dass** sie ein schalenartiges Gestell (9) aufweist, welches eine obere Öffnung bildet, die durch das Tragelement (1) abgedeckt ist.

10. Vorrichtung nach einem der vorangehenden Ansprüche, **dadurch gekennzeichnet, dass** oberhalb des Tragelements (1) mindestens eine Lichtquelle (11) angeordnet ist.
